Europäisches Patentamt

European Patent Office

Office européen des brevets

(19)

(11) Publication number: **0 329 175**
**A1**

(12) **EUROPEAN PATENT APPLICATION**

(21) Application number: 89102795.5

(22) Date of filing: 17.02.89

(51) Int. Cl.⁴: **C12N 15/00 , C12N 1/20 ,
C12P 21/00 , C07K 13/00 ,
//(C12N1/20,C12R1:19)**

---

The microorganism(s) has (have) been deposited with Fermentation Research Institute of the Agency of Industrial Science and Technology under number FERM BP-2332.

(30) Priority: 19.02.88 JP 35042/88

(43) Date of publication of application:
23.08.89 Bulletin 89/34

(84) Designated Contracting States:
BE DE FR GB IT NL

(71) Applicant: **Tosoh Corporation**
**4560, Oaza Tonda**
**Shinnanyo-shi Yamaguchi-ken(JP)**

(72) Inventor: **Ohtsuka, Eiko**
**1422-1-614, 10-jo-nishi 18-chome, Chuo-ku**
**Sapporo-shi Hokkaido(JP)**

(74) Representative: **Patentanwälte Grünecker,
Kinkeldey, Stockmair & Partner
Maximilianstrasse 58
D-8000 München 22(DE)**

(54) **Human nerve growth factor gene segment.**

(57) A novel gene segment that codes for human nerve growth factor and a method of production thereof, a recombinant plasmid containing the gene segment, a microorganism holding the recombinant plasmid in the cells, a method of producing human nerve growth factor by use of the microorganism and a novel human nerve growth factor fused protein which is to be produced by the method.

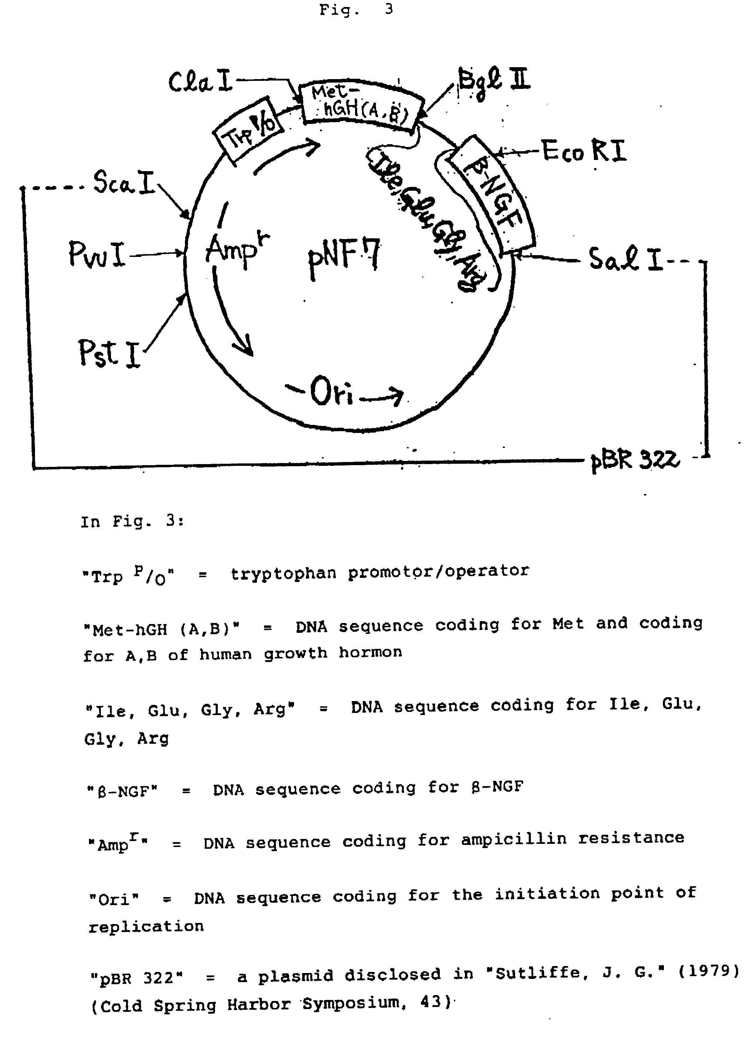

In Fig. 3:

"Trp P/o" - tryptophan promotor/operator

"Met-hGH (A,B)" - DNA sequence coding for Met and coding for A,B of human growth hormon

"Ile, Glu, Gly, Arg" - DNA sequence coding for Ile, Glu, Gly, Arg

"B-NGF" - DNA sequence coding for B-NGF

"Amp^r" - DNA sequence coding for ampicillin resistance

"Ori" - DNA sequence coding for the initiation point of replication

"pBR 322" - a plasmid disclosed in "Sutliffe, J. G." (1979) (Cold Spring Harbor Symposium, 43)

EP 0 329 175 A1

## HUMAN NERVE GROWTH FACTOR GENE SEGMENT

Background of the Invention:

Field of the Invention:

The present invention relates to human nerve growth factor gene segment. More particularly, this invention relates to a novel gene segment that codes for human nerve growth factor and a method of production thereof, a recombinant plasmid containing the gene segment, a microorganism holding the recombinant plasmid in the cells, a method of producing human nerve growth factor by use of the microorganism and a novel human nerve growth factor fused protein which is to be produced by the method.

The nerve growth factor (hereinafter designated as NGF) is an essential substance for the spinal ganglion cells in the viviparity and for the life, growth and differentiation of sympathetic nerve ganglion cells, and is found especially abundantly in the submaxillary gland of male mouse.

Description of the Related Art:

The mouse NGF which has been long investigated is a polypeptide with a sedimentation coefficient 7S and an approximate molecular weight of 131,500 (Biochemistry, vol.6, pp.2203-2209 (967)). A molecule of the 7S NGF consists of 2 $\alpha$-subunits, one $\beta$-subunit and 2$\gamma$-subunits, that is ($\alpha_2\beta\gamma_2$), but among them only the $\beta$-subunit (hereinafter designated as $\beta$-NGF) is responsible to the biological activity as nerve growth factor.

Mouse $\beta$-NGF is a polypeptide consisting of 118 amino acids (Angeletti, K.H. et al.; Proc. Natl. Acad. Sci. USA, vol.68, pp.2417-2420 (1971)). Recently, the amino acid sequence of human $\beta$-NGF was presumed from the cDNA and the similarity to mouse $\beta$-NGF was suggested (Nature, vol.303, pp.821-825 (1983)).

Human nerve growth factor acts. to grow nerve cells such as cells of sensory nerves and sympathetic nerves. More particularly, it functions to grow nerves, polymerize cytoskeletons, increase organelles and participate in the metaplasy of membrane of nuclei. With regard to the anabolism, the human nerve growth factor incorporates precursors of the metabolism such as uridine, glucose and amino acids, and accelerates the syntheses of RNA and protein. As special anabolism, the human nerve growth factor enhances syntheses of tyrosine-hydroxylase, dopamine-$\beta$-hydroxylase and ornithine-decarboxylase. Thus, the human nerve growth factor which holds said properties is recently expected to provide a preventing drug for geriatric dementia.

Nature, vol.303, pp.821-825 (1983) cited above described cloning of human $\beta$-NGF in the pBR 322 plasmid, but did not refer to the expression thereof.

Generally speaking genes of eucaryotes, even if they are integrated in such vectors as to allow expression of procaryote genes and transduced in a procaryotic cell, can not always be expected to be expressed (or to produce a gene product) at least to such a degree as to permit detection. If the base sequence of the genes is made clear, it might be possible to select codons which occur more frequently in microorganisms and to synthesize by chemical synthesis a gene segment which is more easily expressed in microorganism. But the assembly is difficult to realize because of the undesirable annealing which originates from the existence of homologous sequences in the gene sequences concerned, fear of possible loop formation, and further difficult selection of where and how to decide the cite of restriction enzyme when it is integrated in the vectors in the form permitting the expression.

The present inventors already invented, as disclosed in Japanese Laid-Open Patent Application No. Sho 61-205485, methods of producing human nerve growth factor gene segments, recombinant plasmids containing said gene segment, microorganisms holding in its cells said plasmid, and human nerve growth factors from protein accumulated in said microorganism.

However, some problems where found to exist, when a more amount of human nerve growth factor (NGF) was desired. Namely problems are as follows. (i) The rate of expression of NGF in cells is somewhat insufficient when it is expressed and produced directly. (ii) When NGF is expressed and produced in the form of a fused protein, an operation is necessary to cleave the fused protein cite-specifically with an enzyme. Quality of the NGF thus obtained depends seriously on the enzyme employed. The enzyme should be of high quality and readily available at a less cost. The blood coagulation factor factor Xa which is employed in Japanese Laid-Open Patent Application No. Sho 61-205485 can be purified in the procedure as reported in J. Biochem., vol.97, No. 5, P.1347 (1985). But the process is of a small scale and does not afford the amount of factor Xa necessary to perform the present invention.

In this situation, the present inventors continued their efforts in the investigation. They found a method in which a fused protein was expressed via the recognition amino acid sequence of blood coagulation factor thrombin and the fused protein was cleaved to obtain NGF, and this method was more suitable for processes of a large scale. Thus they arrived at completion of the present invention.

Thrombin is an enzyme, one of the blood coagulation factors, obtained by purifying from blood serum as factor Xa is. Since thrombin is now widely used as a drug, high quality products of thrombin are being manufactured on an industrial scale which are more suited for use in the present invention.

Brief Explanation of the Accompanying Drawings:

Fig. 1 is a diagram for illustrating the process of synthesizing DNA and

Fig. 2 is a diagram showing the process for synthesis of the dinucleotide to be used for the synthesis of DNA.

Fig. 3 shows the genetic map of the plasmid pNF-7, and

Fig.4 shows the genetic map of the plasmid pNF-T1.

Detailed Description of the Invention:

The present invention firstly provides human $\beta$-NGF gene segment which ligates in the upstream region a DNA sequence coding for a recognition amino acid sequence for thrombin.

The human $\beta$-NGF gene segment according to the present invention comprises a DNA sequence at the upstream terminal which is expressed by the following formula,

$$5' \quad \begin{matrix} \text{GATCTTCX} \\ \text{AAGX'} \end{matrix} \quad 3' \qquad \ldots \quad (1)$$

(where X is a DNA sequence coding for the recognition amino acid sequence for thrombin starting from an N-terminal and X' is a sequence complementary to X), and a DNA sequence which contains a termination codon at the downstream terminal and terminates at the terminal cleaved by a restriction enzyme which is expressed by the formula,

$$5' \quad \begin{matrix} \text{TY} \\ \text{AY'} \end{matrix} \quad 3' \qquad \ldots \quad (2)$$

(where Y stands for a sequence from the second base in the first termination codon to the 3' terminal, the terminal cleaved by a restriction enzyme, and Y' is a sequence complementary to Y extending to the 5' terminal, the terminal cleaved by a restriction enzyme).

The recognition amino acid sequence for thrombin is exemplified by a sequence Val-Pro-Arg, and an example of X and X' represented by DNA sequences are given by the following formula,

$$5' \quad \begin{matrix} \text{GTTCCGCGT} \\ \text{CAAGGCGCA} \end{matrix} \quad 3' \qquad \ldots \quad (3)$$

The DNA sequences for Y and Y' to be added in the downstream region which contain a termination codon and terminate at a terminal cleaved by a restriction enzyme and exemplified by the following formula which contains a terminal sequence cleaved by a restriction enzyme Sal I,

$$5' \text{ AATAG } 3'$$
$$\text{TTATCAGCT} \quad \ldots (4)$$

In the formula (1) above, the upstream terminal is terminated with the terminal of Bgl II.

The β-NGF gene segment of this invention codes for the same amino acid sequence as the above mentioned cDNA does, excepting that it is substituted by a codon which is expected to be expressed particularly with effect in Escherichia coli.

A recombinant plasmid containing the β-NGF gene segment of this invention can be prepared, for example, by the following process. Namely, starting from the recombinant plasmid pNF-7 which is produced by the method disclosed in "Human nerve growth factor gene segment" in Japanese Laid-Open Patent Application No. Sho 61-205485, a portion of the DNA sequence that codes for the recognition sequence (Ile-Glu-Gly-Arg) of blood coagulation factor Xa represented by the following formula,

$$5' \text{ ATCGAAGGTCGT } 3'$$
$$\text{TAGCTTCCAGCA} \quad \ldots (5)$$

is substituted by a DNA sequence that codes for the recognition sequence (Val-Pro-Arg) of thrombin represented by the following formula,

$$5' \text{ GTTCCGCGT } 3'$$
$$\text{CAAGGCGCA} \quad \ldots (6).$$

More particularly speaking, that site of DNA sequence which codes for the recognition sequence (Ile-Glu-Gly-Arg) for blood coagulation factor Xa of the plasmid pNF-7 is cut out in arbitrary vicinities in the upstream and downstream regions at a recognition sequence of restriction enzyme. Then a new DNA is synthesized in which the portion of the old DNA fragment cut out which codes for the recognition sequence for blood coagulation factor Xa (Ile-Glu-Gly-Arg) is replaced by a DNA sequence which codes for the recognition sequence of thrombin (Val-Pro-Arg), and this new DNA is inserted into the plasmid remaining after the cut-out by a known method.

The restriction enzymes to be employed in this process are exemplified by Bgl II and EcoR I.

When the restriction enzymes, Bgl II and EcoR I, are applied to the recombinant plasmid pNF-7, a DNA having a sequence represented by the following formula is cut out,

$$5' \text{ GATCTTCATCGAAGGTCGTTCCTCCTCTCACCCGATCTTC}$$
$$\text{AAGTAGCTTCCAGCAAGGAGGAGAGTGGGCTAGAAG}$$

$$\text{CACCGTGGCG } 3'$$
$$\text{GTGGCACCGCTTAA} \quad \ldots (7).$$

That portion of the above DNA fragment which codes for the recognition sequence of blood coagulation factor Xa (Ile-Glu-Gly-Arg) is replaced by a DNA sequence which codes for the recognition sequence of thrombin (Val-Pro-Arg), to produce a DNA fragment represented by the following formula,

5' GATCTTCGTTCCGCGTTCCTCCTCTCACCCGATCTTC
AAGCAAGGCGCAAGGAGGAGAGTGGGCTAGAAG

CACCGTGGCG 3'
GTGGCACCGCTTAA                                     . . . (8).

Synthesis of the DNA fragment shown above in the formula (8) is followed by phosphorylation of the 5' terminal with polynucleotidekinase. This may then be inserted in the remainder of pNF-7 from which said DNA fragment has been cut out with T4-Ligase.

In the synthesis of the DNA fragment to be used in this invention, at first some sub-fragments containing about 15 bases are usually synthesized. The sub-fragments are exemplified as follows:

$U_1$ 5'GATCTTCGTTCCGCGTT 3'
$U_2$ 5'CCTCCTCTCACCCGA 3'
$U_3$ 5'TCTCCCACCGTGGCG 3'
$L_1$ 5'GAGGAACGCGGAACGAA 3'
$L_2$ 5'AAGATCGGGTGAGAG 3'
$L_3$ 5'AATTCGCCACGGTGG 3'

These synthesized sub-fragments are mixed together so that a couple of sub-fragments complementary to each other are bonded by the hydrogen bond to form a double strand oligonucleotide. Resulting oligonucleotides are bonded by DNA Ligase to form two complete strands complementary to each other.

At first, the synthesis begins with the preparation of each strand of sub-fragments of the oligonucleotide. The synthesis can be performed, for example, by the solid phase process.

In the solid phase process, preferred carriers are divinylbenzene cross-linked polystyrene resin (refer to K. Miyoshi et al., Nucleic Acids Res., vol.8, p.5507 (1980)) and silica gel (refer to M.D.Matteucci et al., J. Am. Chem. Soc., vol.103, p.3185 (1981)), each having a p-(aminomethyl)phenyl group.

Among four deoxynucleotides which compose 3'-terminals of the fragments, that is deoxycytisine, deoxyadenosine, deoxyguanosine and thymidine, the former three are protected at their amino group with a benzoyl or isobutyryl group and then converted into respective 5'-dimethoxytrityl-3'-succinic acid esters (refer to Broka C. et al., Nucleic Acids Res., vol.8, pp.5461-5471 (1980)).

The above-mentioned carrier having an amino group (nucleoside carrier) is applied to the 3'-terminal of said oligonucleotides. These 3'-terminal nucleoside carriers are treated in two different ways as follows. When the oligonucleotides consist of an even number of deoxyribonucleotides, the mononucleotide existing at the second site from the 3'-terminal (the mononucleotides being protected by N-benzoylation with respect to A and C and by N-isobutylation with respect to G, and each 5'-OH and 3'-phosphoric acid being protected by converting into 4,4'-dimethoxytrityl compound and mono-(o-chlorophenyl)ether, respectively) is combined using a condensation agent. On the other hand, when the oligonucleotides consist of an odd number of deoxyribonucleotides, the dinucleotides (protected in the same manner as mentioned above) existing at the second and third sites from the 3'-terminal are combined using a condensation agent. The condensation agent to be used is appropriately selected from the group consisting of mesitylene sulfonyl-3-nitrotriazolide (MSNT), 2,4,6-triisopropylbenzenesulfonyl-3-nitrotriazolide (TPSNT), mesitylenesulfonyl tetrazolide (MSTe) and 2,4,6-triisopropylbenzenesulfonyl tetrazolide (TPSTe).

With respect to the former oligonucleotides, dinucleotides corresponding to the third and fourth ones which are similarly protected are combined with those corresponding to the fifth and sixth, and so on successively to the direction of 5', while with respect to the latter oligonucleotides dinucleotides corresponding to the fourth and fifth are combined with those corresponding to the sixth and seventh, and so on. However, mononucleotide, trinucleotide and tetranucleotide may be employed if necessary.

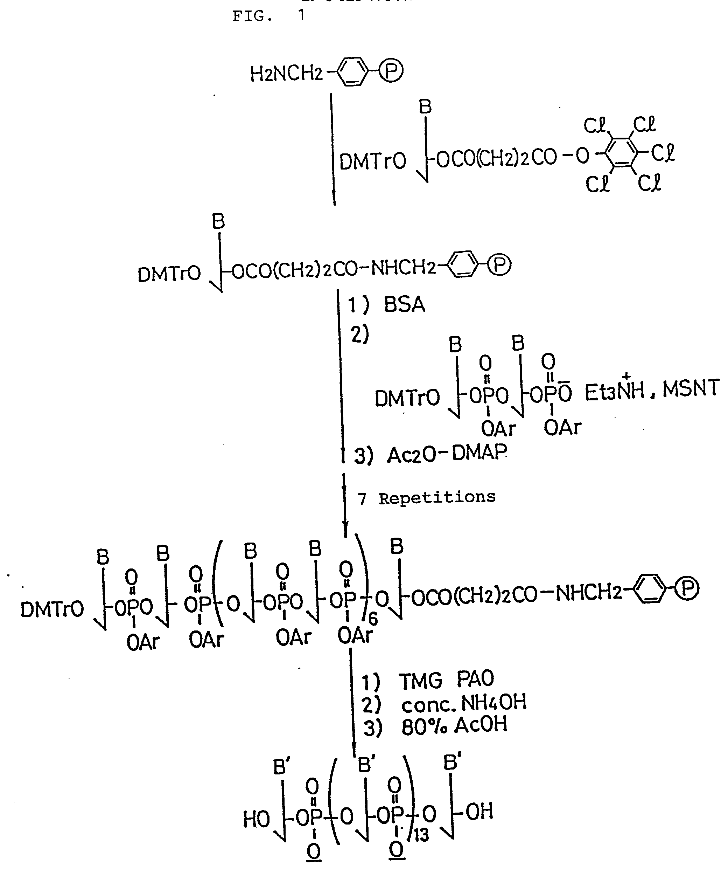

This sequence of preparations is illustrated in Fig. 1. In this figure, P is a copolymer of polystyrene with 1 % of divinylbenzene, bz and ib are benzoyl and isobutyryl groups, respectively, B is bzA, ibG, bzC or T, B' is A, G, C or T, DMTr is 4,4'-dimethoxytrityl group, Ar is o-chlorophenyl group, BSA is a 2 % solution of benzenesulfonic acid in mixed solvents of methylenedichloride and methanol (ratio in volume = 7 : 3), DMAP is a 0.14 M solution of 4-dimethylaminopyridine in pyridine, and TMG-PAO is a 0.5 M solution of 1,1,3,3-tetramethylguanidium-2-pyridine aldoxymate in mixed solvent of dioxane and water (with a ratio in volume of 9 : 1).

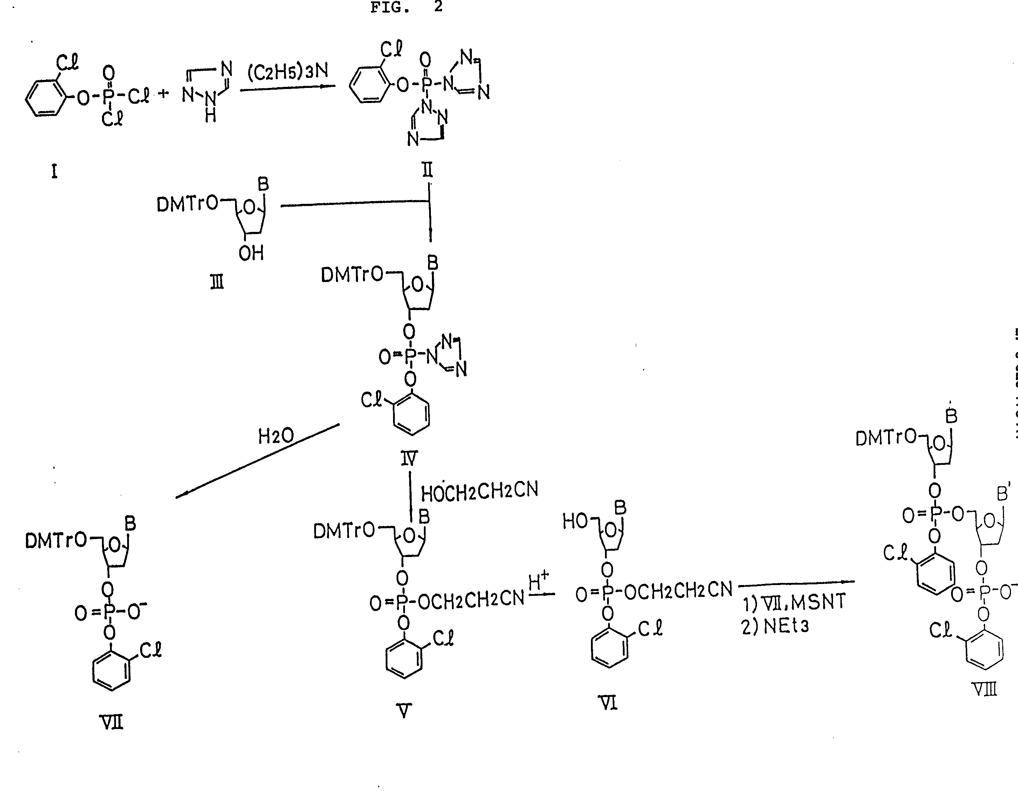

There exist 16 different species in the dinucleotides described above which are composed of 4 nucleotides arranged in different orders. They are synthesized, for example, in a method shown in Fig. 2.

DMTr is 4´,4´-dimethoxytrityl group. B and B´ are selected from the group consisting of 6-N-benzoyladenine-9-yl, 4-N-benzoylcytosine-1-yl, 2-N-isobutylguanine-9-yl, and thymine-1-yl groups and B and B´ may be the same or different from each other. MSNT is mesitylenesulfonyl-3-nitrotriazolide. Protecting groups may be introduced to these dinucleotides readily in a known process (for example, Protein, Nucleic Acid and Enzyme, 26(4), 259 (1981)).

(Plasmid and Preparation thereof)

The present invention provides, in its third item, a recombinant plasmid comprising the β-NGF gene segment of this invention. Namely, it provides a recombinant plasmid which contains β-NGF gene segment and is capable of self-propagation in microorganisms. Preferably the plasmid contains a promoter-operator system which controls the expression of the gene in the upstream region of the human β-NGF gene and, in the domain between the upstream region of the promoter-operator system and the downstream region of the human β-NGF gene, a part to enable the plasmid to self-propagete and a part to afford drug tolerance.

The promoter-operator system is exemplified by a tryptophan promoter-operator system for bacteria which lacks of the attenuating part.

The plasmid of this invention can transform a micro-organism such as Escherichia coli so as to produce a product of human β-NGF gene in the form of a fused protein.

(Transformed Substance)

The plasmid of the present invention can readily transform microorganisms such as Escherichia coli in a conventional manner (for example, Cohen et al., Proc. Natl. Acad. Sci., vol.69, p.2110 (1972)).

Bacteriological properties of these strains are identical to those of host strain, except that the strains have obtained resistance against ampicillin and they produce and express the β-NGF fused protein. The strains can be easily cultured under the same condition as the host strain does, where an ampicillin added culture medium may also be applicable.

The transformed Escherichia coli strain of the present invention, when cultured in a nutrient medium, may propagate human β-NGF genes efficiently. If there exists in the strain a promoter-operator system, particularly tryptophan promoter-operator system lacking of attenuator, which controls and enables the expression of β-NGF of this invention in the upstream region of the β-NGF, can express the human β-NGF in the form of a protein fused with a portion of the human growth hormone at its N-terminal and accumulate it in the body of bacterium. In this case (of the tryptophan promoter-operator system), production of the human β-NGF (or mRNA synthesis) is strongly induced by the addition of indoleacrylic acid.

The fused protein has the following sequence of amino acids,

|  |  |  |  |  |  |  |  |  | 10 |
|---|---|---|---|---|---|---|---|---|---|
| met | phe | pro | thr | ile | pro | leu | ser | arg | leu |
|  |  |  |  |  |  |  |  |  | 20 |
| phe | asp | asn | ala | met | leu | arg | ala | his | arg |
|  |  |  |  |  |  |  |  |  | 30 |
| leu | his | gln | len | ala | phe | asp | thr | tyr | gln |
|  |  |  |  |  |  |  |  |  | 40 |
| glu | phe | glu | glu | ala | tyr | ile | pro | lys | glu |

```
                                                              50
gln   lys   tyr   ser   phe   leu   gln   asn   pro   gln
                                                              60
thr   ser   leu   cys   phe   ser   glu   ser   ile   pro
                                                              70
thr   pro   ser   asn   arg   glu   gln   thr   gln   gln
                                                              80
lys   ser   asn   leu   glu   leu   leu   arg   ile   ser
                                                              90
leu   leu   leu   ile   gln   ser   trp   leu   glu   pro
                                                             100
val   gln   phe   leu   arg   ser   val   phe   ala   asn
                                                             110
ser   leu   val   tyr   gly   ala   ser   asp   ser   asn
                                                             120
val   thr   asp   leu   leu   lys   asp   leu   glu   glu
                                                             130
gly   ile   gln   thr   leu   met   gly   arg   leu   glu
                                                             140
asp   gly   ser   pro   arg   thr   gly   gln   ile   phe


Val   Pro   Arg   SER   SER   SER   HIS   PRO   ILE   PHE
            10
HIS   ARG   GLY   GLU   PHE   SER   VAL   CYS   ASP   SER
            20
VAL   SER   VAL   TRP   VAL   GLY   ASP   LYS   THR   THR
            30
ALA   THR   ASP   ILE   LYS   GLY   LYS   GLU   VAL   MET
```

7

40

VAL   LEU   GLY   GLU   VAL   ASN   ILE   ASN   ASN   SER

50

VAL   PHE   LYS   GLN   TYR   PHE   PHE   GLU   THR   LYS

60

CYS   ARG   ASP   PRO   ASN   PRO   VAL   ASP   SER   GLY

70

CYS   ARG   GLY   ILE   ASP   SER   LYS   HIS   TRP   ASN

80

SER   TYR   CYS   THR   THR   THR   HIS   THR   PHE   VAL

90

LYS   ALA   LEU   THR   MET   ASP   GLY   LYS   GLN   ALA

100

ALA   TRP   ARG   PHE   ILE   ARG   ILE   ASP   THR   ALA

110

CYS   VAL   CYS   VAL   LEU   SER   ARG   LYS   ALA   VAL

118

ARG.

The fused protein accumulated in the bacterial body composed of $\beta$-NGF and a portion of human growth hormone can be separated and recovered, after bacteriolysis of the bacterial body, by the usual method of recovering physiologically active protein. For example, after culturing, bacterial bodies are collected by centrifugation, and treated in a phosphate buffer solution by ultrasonic wave, or with lysozyme, French press and dyno mill. Fractionation with ammonium sulfate or gel filtration of the supernatant liquid gives a protein fraction aimed at. This product may be further purified by use of an antibody column, if a highly pure substance is needed.

The $\beta$-NGF or the protein fused with human growth hormone, when they are adsorbed on cell wall, can be also completely extracted by treating with guanidine hydrochloride, and this is again activated to be recovered with a high yield.

The protein fused with the human growth hormone produced by the microorganism of this invention has the recognition site for thrombin (Val-Pro-Arg) at the site of fusion. By treating with this enzyme, human $\beta$-NGF which is complete and free from additional amino acid residue can be obtained. The treatment may be undergo according to the known process mentioned before, and the product, the human $\beta$-NGF of the natural type, can be purified and separated by the already described procedure.

Detailed Description of the Preferred Embodiments:

The present invention will be explained in more detail. Following reagents are described in abbreviation in the examples.

**Standard buffer** : 50 mM Tris-Hydrochloric acid   pH 7.5
**solution**              35 mM $MgCl_2$

                     35 mM 2-Mercaptoethanol

**Tris-NaCl** : 10 mM Tris-Hydrochloric acid   pH 8.0

9

0.14 mM NaCl

TE-Sucrose : 25 %(w/v) sucrose

50 mM Tris-Hydrochloric acid   pH 8.0

1 mM EDTA

Lysozyme : 10 mg/ml lysozyme

0.25 M Tris-Hydrochloric acid   pH 8.0

0.5 M EDTA : pH 8.0

RNase A : Dissolved in a 0.04 M acetate buffer

solution (pH 5.0) to a concentration

10 mg/ml, and heated at 100°C for

5 min.

Lytic mixture : 0.1 % Non-ionic surfactant

(Triton X-100)

50 mM Tris-Hydrochloric acid   pH 8.0

62.5 mM EDTA

Polyethyleneglycol : Class 1 reagent in powder

(PEG) 6000

5 M NaCl

CsCl : Solid

EB solution : Prepared by dissolving ethidium

bromide in water to 7 mg/ml

TE : 10 mM Tris-Hydrochloric acid   pH 7.4

1.0 mM EDTA

TE-Sarkosyl : 10 mM Tris-Hydrochloric acid

1 mM EDTA

0.38 % Sodium N-Lauroyl Sarkosinate

pH 7.4

TEN : 10 mM Tris-Hydrochloric acid

1 mM EDTA

0.1 M NaCl   pH 7.4

Example 1

(Synthesis of oligonucleotide)

(1) Synthesis of dGATCTTCGTTCCGCGTT (U₁)

5′-(4,4′-Dimethoxytrityl)-N-benzoyldeoxythymidine carrier (50 mg of 100 μmol per gram of the polystyrene resin mentioned before) was kept standing overnight in pyridine at the room temperature and then underwent a condensation reaction in the following procedures.

Procedures:

1) Washing 3 times each with 2 ml of dichloromethanemethanol (7 : 3, V/V).

2) Treatment for 2 min with 2 ml of a 2 % solution of benzenesulfonic acid (in a mixed solvent of dichloromethane and methylalcohol, 7 : 3), followed by washing three times with the same solvent to assure no coloration any more.

3) Washing three times each with 2 ml of pyridine.

4) Adding 0.2 ml of a solution of 5′-(4,4′-dimethoxytrityl)-guanidyl-P-(o-chlorophenyl)-N-benzoyldeoxythymidine-3′-(o-chlorophenyl) phosphate in pyridine (containing 20 mg of nucleotide of said compound) and then distilling the solution under a reduced pressure.

5) Adding 0.2 ml of a pyridine solution containing 20 mg of mesitylenesulfonyl-3-nitrotriazolide as condensation agent and let standing at 40° C for 20 min.

6) Washing twice each with 2 ml of pyridine.

7) Adding 1.8 ml of a 0.1 M solution of dimethylaminopyridine in pyridine and 0.2 ml of acetic anhydride and let standing for 3 min.

8) Washing three times each with 2 ml of pyridine.

Then the same procedures as in 2) and after were followed except using 5′-(4′,4-dimethoxytrityl)-N-benzoyldeoxythymidine-P-(o-chlorophenyl)deoxycytosine-3′-(o-chlorophenyl)phosphate in place of 5′-(4,4′-dimethoxytrityl)-guanidyl-P-(o-chlorophenyl)-N-benzoyldeoxythymidine-3′-(o-chlorophenyl)phosphate. Further, the DNA strand was extended successively to the 5′ direction by use of dCC, dTT, dCG, dTT, dTC and dGA having similar protective groups. Then the resin was immersed in 1 ml of a 0.5 M solution of trimethylguanidium-pyridine-2-aldoxymate in dioxane-water (9 : 1) (C.B.Reese et al., Tetrahedron Lett., 2727 (1978)) and the mixture was shaken for 38 hr. The resin was washed with a 50 % pyridine water and the liquid together with the washing were concentrated under a reduced pressure. The residue mixed with 15 ml of concentrated ammonia water was tightly closed and warmed at 55° C for 5 hr. The ammonia was distilled out, 2 ml of a strongly acid pyridinium type cation ion exchange resin (dowex 50, trade name) was added, the resin was washed with a 50 % pyridine water and the liquid together with the washing were concentrated. A small amount of water was added to the concentrate and the oxime present in it was removed by extraction with ethyl acetate. The aqueous phase was diluted with water and a fraction was taken to quantitatively measure the dimethoxytrityl group for the estimation of the total amount. Assuming the molar extinction coefficient of dimethoxytrityl group be 71.700, a value of $A_{254}$ of 77.4 units suggested that 1 μmol of crude oligonucleotide was synthesized.

The aqueous phase was dried under a reduced pressure. The residue together with 10 ml of 80 % acetic acid was kept at 25° C for 30 min, and then the solvent was distilled out and the residue was dissolved in water and ethyl acetate. The water layer was concentrated and subjected to the ion exchange chromatography. DEAE-Toyopearl 650S (a trade name) was the ion exchanger used. This was placed in a column (0.7 x 21 cm) and eluted with a buffer solution of 7 M urea and 20 mM TRIS-hydrochloric acid (pH = 7.5) under a concentration gradient of sodium chloride of 0.1 to 0.3 M. Oligonucleotide was detected by ultraviolet absorption measurement. The middle portion was taken and dialyzed to remove salt with a cellulose film, to obtain a product (500 $\mu$g) having a $A_{260}$ value of 10.0 units. The purity was ascertained by the HPLC (using a $C_{18}$ silica gel carrier) and the one-dimensional homochromatography giving a single component.

(2) Synthesis of dGAGGAACGCGGAACGAA ($L_1$)

The same procedure for the condensation reaction as in $U_1$ was followed except that a 5'-(4,4-dimethoxytrityl)-adenosine carrier and 5'-(4,4'-dimethoxytrityl)quanidyl-P-(o-chlorophenyl)-N-benzoyldeoxyadenine-3'-(o-chlorophenyl)phosphate were employed at first.

Further employing the dinucleotides which comprised the base sequences according to those in Table 1-2 and protective groups for each, extension of the DNA strand, elimination of the protective groups and separation and purification of the product obtained were carried out in the same manner as in the case for U·, to obtain 5.0 $A_{260}$ units (250 $\mu$g) of $L_1$.

In the similar manner, the oligonucleotides which compose $U_2$, $U_3$, $L_2$ and $L_3$ (see Tables 1-1 and 1-2) were prepared as mentioned below:

A 5'-(4,4'-dimethoxytrityl)-N-isobutyryldeoxyguanosine carrier and a 5'-(4,4'-dimethoxytrityl)-N-benzoyldeoxyadenosine carrier were employed. The dinucleotides which comprised the base sequences according to those in Tables 1-1 and 1-2 and protective groups for each were condensed successively from the 3' terminal to the 5' terminal. Elimination of the protective groups and purification by separation were carried out for the preparation. The oligonucleotides thus prepared are shown in Tables 1-1 and 1-2.

Table 1-1

| $U_1$ | 5'GATCTTCGTTCCGCGTT 3' |
|---|---|
| $U_2$ | 5'CCTCCTCTCACCCGA 3' |
| $U_3$ | 5'TCTCCCACCGTGGCG 3' |

Table 1-2

| $L_1$ | 5'GAGGAACGCGGAACGAA 3' |
|---|---|
| $L_2$ | 5'AAGATCGGGTGAGAG 3' |
| $L_3$ | 5'AATTCGCCACGGTGG 3' |

Example 2

Synthesis of DNA fragment I

The synthesized DNA fragment I was prepared from the oligonucleotides $U_1$, $U_2$ and $U_3$ and $L_1$, $L_2$ and $L_3$ produced by the syntheses of oligonucleotides mentioned above.

A mixture of 1 $\mu$l of a solution containing 0.25 $\mu$Ci of [$\gamma$-$^{32}$P] ATP (1.2 x 10 cpm/$\mu$l), OD 0.2 ($A_{260}$) of each oligonucleotide and 0.5 $\mu$l of a solution of $T_4$ polynucleotide kinase (produced by Takara Shuzo Co., Ltd.) containing 6 u/$\mu$l dissolved in 2 $\mu$l of a kination buffer solution [containing 250 mM of Tris-HCl pH 8.0, 50 mM of $MgCl_2$, 50 mM of 2-mercaptoethanol and 5 mM of spermine] was incubated at 37°C for 20 min. Then 0.025 $\mu$l of 20 mM ATP was added and let to react for 45 min. Further, 1 $\mu$l of 20 mM ATP was added to react for 45 min and then the solution was made 10 $A_{260}$/$\mu$l with added 10 mM Tris-HCl (pH 8.0) and 1 mM EDTA.

For annealing these fragments, the reaction mixture was heated at 70°C for 2 min in a ligation buffer solution (containing 330 mM Tris-HCl pH 7.6, 33 mM of MgCl₂ and 2.5 mM of ATP) and then gradually cooled to the room temperature. The reaction mixture to which 1.3 μl of a 2-mercaptoethanol solution (0.2 M 2-EtSH) was added was cooled to 13°C, then mixed with 2 μl of T₄ DNA ligase (350 u/μl, prepared by Takara Shuzo Co., Ltd.) and incubated for 2 hr. After heating the mixture for 5 min at 65°C, the reaction was stopped. The DNA fragment formed was treated with phenol and mixed with ethanol. Precipitate formed was separated by centrifugation, treated by the electromigration through a 10 % acrylic amide gel (150 V, 2.5 hr), extracted from the gel, precipitated from ethanol, separated by centrifugation and dissolved in a solution of 10 mM Tris-HCl (pH 8.0) and 1 μM EDTA.

Thus, 0.05 A₂₆₀ unit (2.0 μg) of the fragment described below was obtained.

**5'** GATCTTCGTTCCGCGTTCCTCCTCTCACCCGATCTTCCACCGTGGCG **3'**

AAGCAAGGCGCAAGGAGGAGAGTGGGCTAGAAGGTGGCACCGCTTAA

**. . . . Fragment I**

Example 3

Preparation of recombinant and transformed strains

Plasmid pNF-7 (E. coli/pNF-7, deposited at Fermentation Research Institute (FRI), Deposition No. FERM P-8132 of March 8, 1985 - see Fig.3), in an amount of 5 g was suspended in 6 μl of a standard buffer solution and digested at 37°C for 2 hr with 12 u of Bgl II and 10 u of EcoRI. The reaction solution was extracted with phenol, precipitated with ethanol to recover DNA, which was then submitted to electromigration in a 5 % acrylamide gel, to extract a double-stranded DNA portion and to obtain vector DNA pNF-7/Bgl II-EcoR I which was a straight double-stranded DNA weighing about 3 μg. A 3 μl volume of vector DNA pNF-7/Bgl II-EcoR I (0.5 μg/μl) and 3 μl of a kination mixture solution (0.3 μg/μl) of the DNA fragment I synthesized in Example 2 (having a Bgl II site at the 5′ terminal, a succeeding base sequence Val-Pro-Arg of an amino acid codon, a portion of the human nerve growth factor gene and a terminal of the EcoRI site) were mixed with 8 μl of H₂O, 4 μl of a ligation buffer solution and 1 μl of 0.2 M solution of 2-mercaptoethanol. The whole mixture was reacted in the presence of 1 μl of T₄-DNA ligase (350 u/μl) for 20 hr at a temperature of 20°C.

After treating with phenol, DNA was recovered by precipitation with ethanol from the reaction mixture. Transformation and screening followed in the succeeding step of operation.

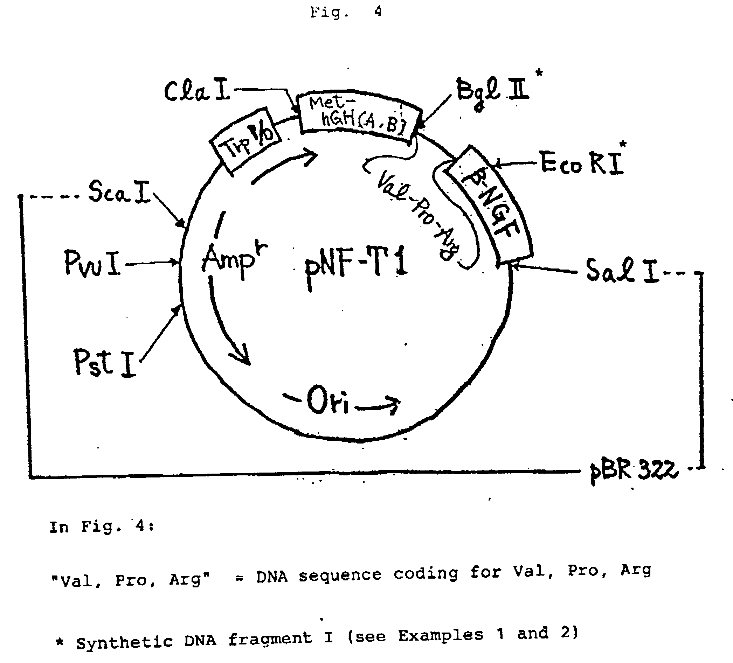

10 Microliters of the plasmid obtained by the above operation and E. coli HB101 were cultured overnight in 5 ml of L broth. Then the culture was repeated using 100 μl of the fresh liquid. When 0.15 A₆₆₀ is reached, this was ice cooled for 30 min. A precipitate obtained by centrifugation was suspended in 50 ml of 50 mM CaCl₂ and ice-cooled for 60 min. The matter obtained by centrifugation was mixed with 0.1 ml of a calcium-treated bacterium which was suspended in a 10 ml solution of 50 mM CaCl₂-20 % glycerol. This was maintained at 0°C, then heated at 42°C for 60 sec and returned to the room temperature. To this was added 1 ml of L-broth (1 g of Bacto-Tryptone, 0.5 g of Yeast Extract, 0.5 g of NaCl and 0.1 g of Glucose dissolved in 100 ml of water and adjusted to pH 7.2) and the mixture was incubated for 30 - 60 min at 37°C. Then followed centrifugation at 13,000 rpm for 5 min. Supernatant liquid was removed and the precipitate formed was suspended in 0.3 ml of L-broth. Each 0.1 ml of the liquid was spread on an agar medium (prepared by adding 1.5 g of agar and 3 mg of ampicillin to a mixture of 1 g of Bacto-Tryptone, 0.5 g of Yeast Extract, 0.5 g of NaCl and 0.1 g of Glucose). Those colonies were selected in which the restriction enzyme cleavage map of the plasmid fraction obtained from each colony agreed with that of the anticipated plasmid (see Fig.4) which is designated as pNF-T1, and as shown in Fig.4, this plasmid has the DNA sequence coding for β-NGF through the DNA sequence coding for the amino acid sequence of Val, Pro, Arg at the 5′ side of the DNA coding hGH (A,B).

The bacteria in the agar medium were suspended in a STET buffer (prepared of 8 % Sucrose, 50 mM Tris-HCl (pH 8.0), 50mM EDTA and 5 % Triton X-100. To this, 20 μl of a lysozyme solution (containing 10 mg lysozyme per ml of 0.25 M Tris-HCl (pH 8.0)) was added, heated in a boiling water for 60 sec and then treated by centrifugation at 13,000 rpm for 10 min.

To the supernatant liquid, 0.5 ml of isopropanol was added and the mixture was kept to stand still at -20°C for 30 min, centrifugated for 10 min at 13,000 rpm and the precipitate formed was dissolved in 50 μl of TE buffer solution. This, mixed with 80 μl of isopropanol, was kept standing at -20°C for 30 min, centrifugated at 13,000 rpm for 10 min and then a precipitate formed was dried.

The precipitate was dissolved in 20.6 μl of $H_2O$, to whcih were added 6 μl of the buffer, 0.9 μl of 5M NaCl, 1 μl of Bgl II (8 u/μl) and 1.5 μl of Sal I (8 u/μl), and the mixture was let to react at 37°C for 90 min. Then this was mixed with 0.2 μl of RNase A (10 mg/ml) and let to react at 37°C for 30 min.

The solution obtained was treated with phenol, precipitated with ethanol, then treated by electrophoresis with 5 % PAGE, to obtain a large amount of DNA aimed at which contained the β-NGF gene segment.

Example 4

(Expression)

E. coli/pNF-T1 which produces β-NGF fused protein as obtained in Example 3 was cultured overnight in a 5 ml of culture medium indicated below,

| $Na_2HPO_4$ | 0.55 g | |
| KH$_2$PO$_4$ | 0.2 g | |
| NaCl | 0.5 g | |
| NH$_4$Cl | 0.1 g | |
| Casamino acid | 0.2 g | |
| Glucose | 0.4 g | |
| MgSO$_4$ | 0.1 mmol | |
| CaCl$_2$ | 0.01 mmol | |
| Thiamin-HCl | 1 mg | |
| Ampicillin | 2 mg | (per 100 ml medium) |

A 100 μl portion of the culture solution obtained above was first cultured in a 5 ml medium consisting of 0.2 % of $MgCl_2$, 0.4 % of casamino acid, glucose-ampicillin (20 μg/ml) and thiamin (10 μg/ml) for an hour, then 20 μl of 3-indolacrylic acid (10 mg/ml ethanol) was added to the medium and the culture continued for additional 24 hr.

After the culturing, bacteria were collected by centrifugation carried out at 13,000 rpm for 5 min.

A portion of the bacteria was suspended in 62.5 ml of a sample buffer Tris-HCl (pH 6.8) containing 2 % of SDS 5 mM EDTA, 10 % of glycerol, 35 mM 2-mercaptoethanol and 0.001 % BPB, heated for 5 min in boiling water and then separated by centrifugation. Electrophoresis on 0.1 % SDS-15 % PAGE was carried out with the supernatant liquid to obtain a matter having a molecular weight $3.0 \times 10^4$.

The pattern was scanned with a densitometer. The amount of formation was calculated. As a result, formation of the hGH-β-NGF fused protein was estimated to amount to 15 % of the total protein.

Example 5

Cleaving NGF from fused protein by thrombin

In 5 l of M9-cas medium, E. coli/pNF-T1 was induced with IAA, cultured for 12 hr, then the bacteria were collected, the total amount weighing about 10 g (wet weight). This was crashed by ultrasonication and separated by centrifugation at 10,000 rpm for 15 min, to obtain a precipitate. The precipitate was dissolved in 50 ml of a solution consisting of 7 M urea, 10 mM Tris-HCl (pH 7.5) and 20 mM mercaptoethanol, and undissolved matter was removed by centrifugation carried out at 4°C and 10,000 rpm for 15 min. The supernatant liquid was made up to 100 ml with the same solution. This was treated with a DEAE-cellulose column (2.8 x 15 cm φ) equilibrated with the same solution, applying the linear concentration gradient elution technique by use of 0 to 1.3 M sodium chloride solutions as eluent (1 liter in total). Protein which

14

was eluted was taken in fractions, each fraction being of a 7 ml volume. Detection of the substance aimed at was conducted with each fraction with SDS-12.5 % PAGE, and dialysed to water, to obtain about 12 mg of fused protein. In 100 mM Tris-HCl (pH 8.0), about 80 μg of this substance was hydrolyzed with 1 μg of thrombin at 37° C for 1.5 hr, the decomposition product by hydrolysis was submitted to the electrophoresis separation with acrylic amide, to obtain a molecular weight fraction corresponding to 13,000 which was estimated to be human $\beta$-NGF.

The hydrolysis product, in the NGF activity test using rat pheochromocytoma pc 12 cells, proved to be active.

(Effect of the Invention)

The $\beta$-NGF gene segment of the present invention is readily expressable in E. coli and easy in preparation.

The recombinant plasmid of this invention is capable of effective multiplication of said $\beta$-NGF gene segment. Further by transducing an appropriate expressing system, those microorganisms which were transformed by the plasmid can be given the activity of producing $\beta$-NGF fused protein.

Such $\beta$-NGF fused protein permits to produce the human $\beta$-NGF by use of blood coagulation factor thrombin which is manufactured on an large scale, and so,is more suited for industrial use than other enzyme such as Factor Xa.

Furthermore, the present invention permits the human $\beta$-NGF which is not produced in nature to be produced effectively and abundantly. This leads to its utilization as a drug. Thus, the present invention promises novel therapeutics to various nervous diseases, including dementia of the aged, by use of human nerve growth factors which can be produced in a large scale by the present invention.

## Claims

1. Human nerve growth factor gene segment comprising a DNA sequence, at the upstream terminal of the human nerve growth factor gene, which is expressed by the formula,

$$5'\text{ GATCTTCX }3'$$
$$\text{AAGX}'$$

(where X is a DNA sequence coding for a recognition amino acid sequence for thrombin, one of blood coagulation factors, and X' is a sequence complementary to X), and
a DNA sequence which contains a termination codon at the downstream terminal and terminates at the terminal cleaved by a restriction enzyme which is expressed by the formula,

$$5'\text{ TY }3'$$
$$\text{AY}'$$

(where Y stands for a sequence from the second base in the first termination codon to the 3' terminal, the terminal cleaved by a restriction enzyme, and Y' is a sequence complementary to Y extending to the 5' terminal, the terminal cleaved by a restriction enzyme).

2. Human nerve growth factor gene segment according to claim 1, in which X in the DNA sequence to be added in the upstream region of the human nerve growth factor gene in a DNA sequence that codes for an amino acid sequence represented as Val-Pro-Arg from the N-terminal in the recognition amino acid sequence for thrombin as one of the blood coagulation factors, and X' is a sequence complementary to X.

3. Human nerve growth factor gene segment according to claim 1 or 2, in which X and X' in the DNA sequence to be added in the upstream region of the human nerve growth factor gene are sequences expressed by the formula,

5' GTTCCGCGT 3'
    CAAGGCGCA .

4. Human nerve growth factor gene segment according to claim 1, in which Y and Y' in the DNA sequence to be added in the downstream region of the human nerve growth factor gene are sequences expressed by the formula,

5' AATAG 3'
    TTATCAGCT .

5. Human nerve growth factor gene segment according to any of claims 1 through 4, in which the DNA sequence of the human nerve growth factor gene is a DNA sequence coding for an amino acid sequence expressed by the formula,

|  |  |  |  |  |  |  |  |  | 10 |
|---|---|---|---|---|---|---|---|---|---|
| SER | SER | SER | HIS | PRO | ILE | PHE | HIS | ARG | GLY |
|  |  |  |  |  |  |  |  |  | 20 |
| GLU | PHE | SER | VAL | CYS | ASP | SER | VAL | SER | VAL |
|  |  |  |  |  |  |  |  |  | 30 |

| TRP | VAL | GLY | ASP | LYS | THR | THR | ALA | THR | ASP |
| | | | | | | | | | 40 |
| ILE | LYS | GLY | LYS | GLU | VAL | MET | VAL | LEU | GLY |
| | | | | | | | | | 50 |
| GLU | VAL | ASN | ILE | ASN | ASN | SER | VAL | PHE | LYS |
| | | | | | | | | | 60 |
| GLN | TYR | PHE | PHE | GLU | THR | LYS | CYS | ARG | ASP |
| | | | | | | | | | 70 |
| PRO | ASN | PRO | VAL | ASP | SER | GLY | CYS | ARG | GLY |
| | | | | | | | | | 80 |
| ILE | ASP | SER | LYS | HIS | TRP | ASN | SER | TYR | CYS |
| | | | | | | | | | 90 |
| THR | THR | THR | HIS | THR | PHE | VAL | LYS | ALA | LEU |
| | | | | | | | | | 100 |
| THR | MET | ASP | GLY | LYS | GLN | ALA | ALA | TRP | ARG |
| | | | | | | | | | 110 |
| PHE | ILE | ARG | ILE | ASP | THR | ALA | CYS | VAL | CYS |
| | | | | | | | 118 | | |
| VAL | LEU | SER | ARG | LYS | ALA | VAL | ARG. | | |

6. Human nerve growth factor gene segment according to claim 5, the DNA sequence of the human nerve growth factor gene is expressed by the formula,

5'
TCCTCCTCTCACCCGATCTTCCACCGTGGC
AGGAGGAGAGTGGGCTAGAAGGTGGCACCG

GAATTCTCTGTTTGCGATTCCGTTTCTGTA
CTTAAGAGACAAACGCTAAGGCAAAGACAT

TGGGTTGGTGACAAAACCACTGCTACCGAC
ACCCAACCACTGTTTTGGTGACGATGGCTG

ATCAAAGGTAAAGAAGTAATGGTTCTGGGC
TAGTTTCCATTTCTTCATTACCAAGACCCG

GAAGTTAACATTAATAACTCCGTATTCAAG
CTTCAATTGTAATTATTGAGGCATAAGTTC

CAATATTTTTTTGAAACTAAATGCCGTGAC
GTTATAAAAAAACTTTGATTTACGGCACTG

CCGAACCCGGTTGACTCTGGTTGTCGTGGT
GGCTTGGGCCAACTGAGACCAACAGCACCA

ATCGACTCCAAACACTGGAACTCTTACTGC
TAGCTGAGGTTTGTGACCTTGAGAATGACG

ACCACTACCCACACCTTCGTGAAAGCTCTG
TGGTGATGGGTGTGGAAGCACTTTCGAGAC

ACAATGGATGGTAAACAGGCAGCTTGGCGT
TGTTACCTACCATTTGTCCGTCGAACCGCA

TTCATCCGCATCGACACCGCTTGCGTATGT
AAGTAGGCGTAGCTGTGGCGAACGCATACA

GTTCTGTCTCGTAAGGCTGTTCGT $^{3'}$
CAAGACAGAGCATTCCGACAAGCA .

7. Recombinant plasmid which is capable of self-propagation in microorganisms comprising a human nerve growth factor gene segment having a DNA sequence, at the upstream terminal of the human nerve growth factor gene, which is expressed by the formula,

$$5' \text{GATCTTCX} \ 3'$$
$$\text{AAGX}'$$

(where X is a DNA sequence coding for a recognition amino aid sequence for thrombin, one of blood coagulation factors, and X′ is a sequence complementary to X), and a DNA sequence which contains a termination codon at the downstream terminal and terminates at the terminal cleaved by a restriction enzyme expressed by the formula,

$$5' \text{TY} \ 3'$$
$$\text{AY}'$$

(where Y stands for a sequence from the second base on the first termination codon to the 3′ terminal, the terminal cleaved by a restriction enzyme, and Y′ is a sequence complementary to Y extending to the 5′ terminal, the terminal cleaved by a restriction enzyme).

8. Recombinant plasmid according to claim 7, which further comprises at a further upstream region of the human nerve growth factor gene segment, a promoter-operator system which enables the expression of the DNA and, in the region between the upstream region of the promoter-operator system and the downstream region of the human nerve growth gene, a part which enables self-propagation of the recombinant plasmid and a part coding drug tolerance.

9. Recombinant plasmid according to claim 8, in which the promoter-operator system is a bacterial promoter-operator system for tryptophan which lacks the attenuating part.

10. Recombinant plasmid according to claim 7, in which X in the DNA sequence added to the upstream region of the human nerve growth factor gene is a DNA sequence coding for an amino acid sequence which is represented as Val-Pro-Arg from the N-terminal and $X'$ is a sequence complementary to X.

11. Recombinant plasmid according to claim 10, in which X and $X'$ in the DNA sequence to be added in the upstream region of the human nerve growth factor gene are sequences expressed by the formula,

$$5'\ \text{GTTCCGCGT}\ 3'$$
$$\text{CAAGGCGCA} \ .$$

12. Recombinant plasmid according to claim 7, in which Y and $Y'$ in the DNA sequence to be added in the upstream region of the human nerve growth factor gene are expressed by the formula,

$$5'\ \text{AATAG}\ 3'$$
$$\text{TTATCAGCT} \ .$$

13. Recombinant plasmid according to any of claims 7 through 12, in which the DNA sequence of the human nerve growth factor gene is a DNA sequence coding for an amino acid sequence represented by the formula,

|  |  |  |  |  |  |  |  |  | 10 |
|---|---|---|---|---|---|---|---|---|---|
| SER | SER | SER | HIS | PRO | ILE | PHE | HIS | ARG | GLY |

|  |  |  |  |  |  |  |  |  | 20 |
|---|---|---|---|---|---|---|---|---|---|
| GLU | PHE | SER | VAL | CYS | ASP | SER | VAL | SER | VAL |

|  |  |  |  |  |  |  |  |  | 30 |
|---|---|---|---|---|---|---|---|---|---|
| TRP | VAL | GLY | ASP | LYS | THR | THR | ALA | THR | ASP |

ILE LYS GLY LYS GLU VAL MET VAL LEU GLY (40)

GLU VAL ASN ILE ASN ASN SER VAL PHE LYS (50)

GLN TYR PHE PHE GLU THR LYS CYS ARG ASP (60)

PRO ASN PRO VAL ASP SER GLY CYS ARG GLY (70)

ILE ASP SER LYS HIS TRP ASN SER TYR CYS (80)

THR THR THR HIS THR PHE VAL LYS ALA LEU (90)

THR MET ASP GLY LYS GLN ALA ALA TRP ARG (100)

PHE ILE · ARG ILE ASP THR ALA CYS VAL CYS (110)

VAL LEU SER ARG LYS ALA VAL ARG. (118)

14. Recombinant plasmid according to claim 13, in which the DNA sequence of the human nerve growth factor gene is expressed by the formula,

5' TCCTCCTCTCACCCGATCTTCCACCGTGGC
AGGAGGAGAGTGGGCTAGAAGGTGGCACCG

GAATTCTCTGTTTGCGATTCCGTTTCTGTA
CTTAAGAGACAAACGCTAAGGCAAAGACAT

TGGGTTGGTGACAAAACCACTGCTACCGAC
ACCCAACCACTGTTTTGGTGACGATGGCTG

ATCAAAGGTAAAGAAGTAATGGTTCTGGGC
TAGTTTCCATTTCTTCATTACCAAGACCCG

20

GAAGTTAACATTAATAACTCCGTATTCAAG
CTTCAATTGTAATTATTGAGGCATAAGTTC

CAATATTTTTTTGAAACTAAATGCCGTGAC
GTTATAAAAAAACTTTGATTTACGGCACTG

CCGAACCCGGTTGACTCTGGTTGTCGTGGT
GGCTTGGGCCAACTGAGACCAACAGCACCA

ATCGACTCCAAACACTGGAACTCTTACTGC
TAGCTGAGGTTTGTGACCTTGAGAATGACG

ACCACTACCCACACCTTCGTGAAAGCTCTG
TGGTGATGGGTGTGGAAGCACTTTCGAGAC

ACAATGGATGGTAAACAGGCAGCTTGGCGT
TGTTACCTACCATTTGTCCGTCGAACCGCA

TTCATCCGCATCGACACCGCTTGCGTATGT
AAGTAGGCGTAGCTGTGGCGAACGCATACA

GTTCTGTCTCGTAAGGCTGTTCGT $^{3'}$
CAAGACAGAGCATTCCGACAAGCA .

15. Recombinant plasmid according to any of claims 7 through 14, in which the DNA sequence of the promoter-operator system from the transcription initiation point of messenger RNA to the human nerve growth factor gene comprises a part of a DNA sequence which codes for a human growth hormone.

16. Recombinant plasmid according to claim 15, in which a sequence represented by the following formula,

$^{5'}$ AAGTTCACGTAAAAAGGGTAATCGATATGT
TTCAAGTGCATTTTTCCCATTAGCTATACA

TCCCAACTATTCCACTGAGTCGCCTGTTCG
AGGGTTGATAAGGTGACTCAGCGGACAAGC

```
ATAACGCGATGCTGCGTGCGCATCGTCTGC
TATTGCGCTACGACGCACGCGTAGCAGACG


ACCAACTGGCTTTCGACACTTACCAGGAGT
TGGTTGACCGAAAGCTGTGAATGGTCCTCA


TCGAAGAAGCATACATCCCGAAAGAACAGA
AGCTTCTTCGTATGTAGGGCTTTCTTGTCT


AATACAGCTTCCTTCAGAACCCACAGACCT
TTATGTCGAAGGAAGTCTTGGGTGTCTGGA


CGTTGTGTTTCTCTGAAAGTATCCCGACCC
GCAACACAAAGAGACTTTCATAGGGCTGGG


CTTCTAACCGCGAAGAGACCCAGCAGAAAT
GAAGATTGGCGCTTCTCTGGGTCGTCTTTA


CGAACCTTGAACTGCTTCGTATCTCGCTGC
GCTTGGAACTTGACGAAGCATAGAGCGACG


TTCTCATTCAGTCGTGGCTGGAGCCAGTAC
AAGAGTAAGTCAGCACCGACCTCGGTCATG


AGTTCCTGCGTTCGGTTTTCGCAAACTCAC
TCAAGGACGCAAGCCAAAAGGGTTTGAGTG


TGGTATACGGTGCGTCTGACAGTAACGTTT
ACCATATGCCACGCAGACTGTCATTGCAAA


ACGACCTGCTGAAAGACCTTGAAGAAGGGA
TGCTGGACGACTTTCTGGAACTTCTTCCCT


TCCAGACCCTGATGGGTCGCCTGGAAGATG
AGGTCTGGGACTACCCAGCGGACCTTCTAC


GTTCACCACGCACTGGTCAGATCTTC ³'
CAAGTGGTGCGTGACCAGTCTAGAAG
```

is comprised as a part of the DNA sequence which codes for the human growth hormone.

17. Microorganism of Escherichia genus holding in its cells a recombinant plasmid which is capable of self-propagation in a microorganism comprising human nerve growth factor gene segment having a DNA sequence, at the upstream terminal of the human nerve growth factor gene, which is expressed by the formula,

$$5' \quad GATCTTCX \quad 3'$$
$$AAGX'$$

(where X is a DNA sequence coding for a recognition amino acid sequence for thrombin, one of blood coagulation factors, and $X'$ is a sequence complementary to X), and
a DNA sequence which contains a termination codon at the downstream terminal and terminates at the terminal cleaved by a restriction enzyme expressed by the formula,

$$5'\,TY\,3'$$
$$AY'$$

(where Y stands for a sequence from the second base on the first termination codon in the $3'$ terminal, the terminal cleaved by a restriction enzyme, and $Y'$ is a sequence complementary to Y extending to the $5'$ terminal, the terminal cleaved by a restriction enzyme).

18. Microorganism according to claim 17, in which further comprises in a further upstream region of the human nerve growth factor gene segment, a promoter-operator system which enables the expression of the DNA and, in the region between the upstream region of the promoter-operator system and the downstream region of the human nerve growth gene, a part which enables self-propagation of the recombinant plasmid and a part coding drug tolerance.

19. Microorganism described according to claim 18, in which the promoter-operator system is a bacterial promoter-operator system for tryptophan which lacks the attenuating part.

20. Microorganism according to claim 17 holding in its cells a recombinant plasmid in which X in the DNA sequence added to the upstream region of the human nerve growth factor gene is a DNA sequence coding for an amino acid sequence which is represented as Val-Pro-Arg from the N-terminal and $X'$ is a sequence complementary to X.

21. Microorganism according to claim 20 holding in its cells a recombinant plasmid in which X and $X'$ in the DNA sequence to be added in the upstream region of the human nerve growth factor gene are sequences expressed by the formula,

$$5'\,GTTCCGCGT\,3'$$
$$CAAGGCGCA\,.$$

22. Microorganism according to claim 17 holding in its cells a recombinant plasmid in which Y and $Y'$ in the DNA sequence to be added in the downstream region of the human nerve growth factor gene are expressed by the formula,

$$5'\,AATAG\,3'$$
$$TTATCAGCT\,.$$

23. Microorganism according to any of claims 17 through 22 holding in its cells a recombinant plasmid in which the DNA sequence of the human nerve growth factor gene is a DNA sequence coding for an amino acid sequence represented by the formula,

.

```
                                                            10
SER   SER   SER   HIS   PRO   ILE   PHE   HIS   ARG   GLY

                                                            20
GLU   PHE   SER   VAL   CYS   ASP   SER   VAL   SER   VAL

                                                            30
TRP   VAL   GLY   ASP   LYS   THR   THR   ALA   THR   ASP

                                                            40
ILE   LYS   GLY   LYS   GLU   VAL   MET   VAL   LEU   GLY

                                                            50
GLU   VAL   ASN   ILE   ASN   ASN   SER   VAL   PHE   LYS

                                                            60
GLN   TYR   PHE   PHE   GLU   THR   LYS   CYS   ARG   ASP

                                                            70
PRO   ASN   PRO   VAL   ASP   SER   GLY   CYS   ARG   GLY

                                                            80
ILE   ASP   SER   LYS   HIS   TRP   ASN   SER   TYR   CYS

                                                            90
THR   THR   THR   HIS   THR   PHE   VAL   LYS   ALA   LEU

                                                           100
THR   MET   ASP   GLY   LYS   GLN   ALA   ALA   TRP   ARG


                                                           110
PHE   ILE   ARG   ILE   ASP   THR   ALA   CYS   VAL   CYS

                                                   118
VAL   LEU   SER   ARG   LYS   ALA   VAL   ARG.
```

24. Microorganism according to claim 23 holding in its cells a recombinant plasmid in which the DNA sequence of the human nerve growth factor gene is expressed by the formula,

5' TCCTCCTCTCACCCGATCTTCCACCGTGGC
AGGAGGAGAGTGGGCTAGAAGGTGGCACCG

GAATTCTCTGTTTGCGATTCCGTTTCTGTA
CTTAAGAGACAAACGCTAAGGCAAAGACAT

TGGGTTGGTGACAAAACCACTGCTACCGAC
ACCCAACCACTGTTTTGGTGACGATGGCTG

ATCAAAGGTAAAGAAGTAATGGTTCTGGGC
TAGTTTCCATTTCTTCATTACCAAGACCCG

GAAGTTAACATTAATAACTCCGTATTCAAG
CTTCAATTGTAATTATTGAGGCATAAGTTC

CAATATTTTTTTGAAACTAAATGCCGTGAC
GTTATAAAAAAACTTTGATTTACGGCACTG

CCGAACCCGGTTGACTCTGGTTGTCGTGGT
GGCTTGGGCCAACTGAGACCAACAGCACCA

ATCGACTCCAAACACTGGAACTCTTACTGC
TAGCTGAGGTTTGTGACCTTGAGAATGACG

ACCACTACCCACACCTTCGTGAAAGCTCTG
TGGTGATGGGTGTGGAAGCACTTTCGAGAC

ACAATGGATGGTAAACAGGCAGCTTGGCGT
TGTTACCTACCATTTGTCCGTCGAACCGCA

TTCATCCGCATCGACACCGCTTGCGTATGT
AAGTAGGCGTAGCTGTGGCGAACGCATACA

GTTCTGTCTCGTAAGGCTGTTCGT 3'
CAAGACAGAGCATTCCGACAAGCA .

25. Microorganism according to any of claims 17 through 24 holding in its cells a recombinant plasmid in which the DNA sequence of the promoter-operator system from the transcription initiation point of messenger RNA to the human nerve growth factor gene comprises a part of a DNA sequence which codes for a human growth hormone.

26. Microorganism according to claims 25 holding in its cells a recombinant plasmid in which a sequence represented by the following formula,

5′ AAGTTCACGTAAAAAGGGTAATCGATATGT
TTCAAGTGCATTTTTCCCATTAGCTATACA

TCCCAACTATTCCACTGAGTCGCCTGTTCG
AGGGTTGATAAGGTGACTCAGCGGACAAGC

ATAACGCGATGCTGCGTGCGCATCGTCTGC
TATTGCGCTACGACGCACGCGTAGCAGACG

ACCAACTGGCTTTCGACACTTACCAGGAGT
TGGTTGACCGAAAGCTGTGAATGGTCCTCA

TCGAAGAAGCATACATCCCGAAAGAACAGA
AGCTTCTTCGTATGTAGGGCTTTCTTGTCT

AATACAGCTTCCTTCAGAACCCACAGACCT
TTATGTCGAAGGAAGTCTTGGGTGTCTGGA

CGTTGTGTTTCTCTGAAAGTATCCCGACCC
GCAACACAAAGAGACTTTCATAGGGCTGGG

CTTCTAACCGCGAAGAGACCCAGCAGAAAT
GAAGATTGGCGCTTCTCTGGGTCGTCTTTA

CGAACCTTGAACTGCTTCGTATCTCGCTGC
GCTTGGAACTTGACGAAGCATAGAGCGACG

TTCTCATTCAGTCGTGGCTGGAGCCAGTAC
AAGAGTAAGTCAGCACCGACCTCGGTCATG

AGTTCCTGCGTTCGGTTTTCGCAAACTCAC
TCAAGGACGCAAGCCAAAAGGGTTTGAGTG

TGGTATACGGTGCGTCTGACAGTAACGTTT
ACCATATGCCACGCAGACTGTCATTGCAAA

ACGACCTGCTGAAAGACCTTGAAGAAGGGA
TGCTGGACGACTTTCTGGAACTTCTTCCCT

TCCAGACCCTGATGGGTCGCCTGGAAGATG
AGGTCTGGGACTACCCAGCGGACCTTCTAC

GTTCACCACGCACTGGTCAGATCTTC 3′
CAAGTGGTGCGTGACCAGTCTAGAAG

constitutes a part of the DNA sequence which codes for the human growth hormone.

27. Method for production of a human nerve growth factor comprising culturing in a nutrient culture medium a microorganism of Escherichia genus holding in its cells a recombinant plasmid, which is capable of self-propagation in the microorganism and of expressing protein fused with human nerve growth factor, containing a human nerve growth factor gene segment having a DNA sequence, at the upstream terminal of the human nerve growth factor gene, which is expressed by the formula,

$$5' \overset{\cdot}{GATCTTCX} \; 3'$$
$$AAGX'$$

(where X is a DNA sequence coding for a recognition amino acid sequence for thrombin, one of blood coagulation factors, and X' is a sequence complementary to X), and

a DNA sequence which contains a termination codon at the downstream terminal and terminates at the terminal cleaved by a restriction enzyme expressed by the formula,

$$5' \; TY \; 3'$$
$$AY'$$

(where Y stands for a sequence from the second base on the first termination codon to the 3' terminal, the terminal cleaved by a restriction enzyme, and Y' is a sequence complementary to Y extending to the 5' terminal, the terminal cleaved by a restriction enzyme), and then collecting the protein fused with human nerve growth factor that is accumulated, followed by digesting the same with thrombin.

28. Method according to claim 27, further comprising use of a microorganism holding in its cells a plasmid which contains, in a further upstream region of the human nerve growth factor gene segment, a promoter-operator system for tryptophan in bacteria lacking the attenuating part and, in the region between the upstream region of the promoter-operator system and the downstream region of the human nerve growth gene, a part which enables self-propagation of the recombinant plasmid and a part coding the drug tolerance.

29. Method according to claim 27, further comprising use of a microorganism that holds a recombinant plasmid carrying a human nerve growth factor gene in which X in the DNA sequence added to the upstream region of the human nerve growth factor gene of the recombinant plasmid is a DNA sequence coding for an amino acid sequence represented as Val-Pro-Arg from the N-terminal in the recognition amino acid sequence for thrombin, one of the blood coagulation factors, and X' is a sequence complementary to X.

30. Method according to claim 29, in which employed is a microorganism that holds a recombinant plasmid having a human nerve growth factor gene segment where X and X' in the DNA sequence added in the upstream region of human nerve growth factor gene are the sequences expressed by the formula,

$$5' \; GTTCCGCGT \; 3'$$
$$CAAGGCGCA \; .$$

31. Method according to claim 27, in which employed is a microorganism carrying a recombinant plasmid that holds a human nerve growth factor gene segment in which Y and Y' in the DNA sequence to be added in the downstream region of the human nerve growth factor gene are sequences expressed by the formula,

$$5' \; AATAG \; 3'$$
$$TTATCAGCT \; .$$

32. Method according to any of claims 27 through 31, in which employed is a microorganism holding a recombinant plasmid in which a DNA sequence of the human nerve growth factor gene is a DNA sequence coding for an amino acid sequence represented by the formula,

```
                                                          10
    SER   SER   SER   HIS   PRO   ILE   PHE   HIS   ARG   GLY
                                                          20
    GLU   PHE   SER   VAL   CYS   ASP   SER   VAL   SER   VAL
                                                          30
    TRP   VAL   GLY   ASP   LYS   THR   THR   ALA   THR   ASP
                                                          40
    ILE   LYS   GLY   LYS   GLU   VAL   MET   VAL   LEU   GLY
                                                          50
    GLU   VAL   ASN   ILE   ASN   ASN   SER   VAL   PHE   LYS
                                                          60
    GLN   TYR   PHE   PHE   GLU   THR   LYS   CYS   ARG   ASP
                                                          70
    PRO   ASN   PRO   VAL   ASP   SER   GLY   CYS   ARG   GLY
                                                          80
    ILE   ASP   SER   LYS   HIS   TRP   ASN   SER   TYR   CYS
                                                          90
    THR   THR   THR   HIS   THR   PHE   VAL   LYS   ALA   LEU
                                                          100
    THR   MET   ASP   GLY   LYS   GLN   ALA   ALA   TRP   ARG
                                                          110
    PHE   ILE   ARG   ILE   ASP   THR   ALA   CYS   VAL   CYS

                                                          118
        VAL   LEU   SER   ARG   LYS   ALA   VAL   ARG.
```

33. Method according to claim 32, in which employed is a microorganism holding a recombinant plasmid containing a human nerve growth factor gene sequence in which a DNA sequence of the human nerve growth factor gene is expressed by the formula,

28

5'
TCCTCCTCTCACCCGATCTTCCACCGTGGC
AGGAGGAGAGTGGGCTAGAAGGTGGCACCG

GAATTCTCTGTTTGCGATTCCGTTTCTGTA
CTTAAGAGACAAACGCTAAGGCAAAGACAT

TGGGTTGGTGACAAAACCACTGCTACCGAC
ACCCAACCACTGTTTTGGTGACGATGGCTG

ATCAAAGGTAAAGAAGTAATGGTTCTGGGC
TAGTTTCCATTTCTTCATTACCAAGACCCG

GAAGTTAACATTAATAACTCCGTATTCAAG
CTTCAATTGTAATTATTGAGGCATAAGTTC

CAATATTTTTTTGAAACTAAATGCCGTGAC
GTTATAAAAAAACTTTGATTTACGGCACTG

CCGAACCCGGTTGACTCTGGTTGTCGTGGT
GGCTTGGGCCAACTGAGACCAACAGCACCA

ATCGACTCCAAACACTGGAACTCTTACTGC
TAGCTGAGGTTTGTGACCTTGAGAATGACG

ACCACTACCCACACCTTCGTGAAAGCTCTG
TGGTGATGGGTGTGGAAGCACTTTCGAGAC

ACAATGGATGGTAAACAGGCAGCTTGGCGT
TGTTACCTACCATTTGTCCGTCGAACCGCA

TTCATCCGCATCGACACCGCTTGCGTATGT
AAGTAGGCGTAGCTGTGGCGAACGCATACA

GTTCTGTCTCGTAAGGCTGTTCGT 3'
CAAGACAGAGCATTCCGACAAGCA .

34. Method according to any of claims 27 through 33, further comprising employing a microorganism that carries a recombinant plasmid holding a human nerve growth factor gene segment in which a DNA sequence of a promoter-operator system extending from the transcription initiation point of messenger RNA to the human nerve growth factor gene contains a part of a DNA sequence which codes for a human growth hormone.

35. Method according to claim 27, further comprising employing a microorganism that carries a recombinant plasmid holding a human nerve growth factor gene segment in which a sequence represented by the following formula,

5'AAGTTCACGTAAAAAGGGTAATCGATATGT
TTCAAGTGCATTTTTCCCATTAGCTATACA

TCCCAACTATTCCACTGAGTCGCCTGTTCG
AGGGTTGATAAGGTGACTCAGCGGACAAGC

ATAACGCGATGCTGCGTGCGCATCGTCTGC
TATTGCGCTACGACGCACGCGTAGCAGACG

ACCAACTGGCTTTCGACACTTACCAGGAGT
TGGTTGACCGAAAGCTGTGAATGGTCCTCA

TCGAAGAAGCATACATCCCGAAAGAACAGA
AGCTTCTTCGTATGTAGGGCTTTCTTGTCT

AATACAGCTTCCTTCAGAACCCACAGACCT
TTATGTCGAAGGAAGTCTTGGGTGTCTGGA

CGTTGTGTTTCTCTGAAAGTATCCCGACCC
GCAACACAAAGAGACTTTCATAGGGCTGGG

CTTCTAACCGCGAAGAGACCCAGCAGAAAT
GAAGATTGGCGCTTCTCTGGGTCGTCTTTA

CGAACCTTGAACTGCTTCGTATCTCGCTGC
GCTTGGAACTTGACGAAGCATAGAGCGACG

TTCTCATTCAGTCGTGGCTGGAGCCAGTAC
AAGAGTAAGTCAGCACCGACCTCGGTCATG

AGTTCCTGCGTTCGGTTTTCGCAAACTCAC
TCAAGGACGCAAGCCAAAAGGGTTTGAGTG

TGGTATACGGTGCGTCTGACAGTAACGTTT
ACCATATGCCACGCAGACTGTCATTGCAAA

ACGACCTGCTGAAAGACCTTGAAGAAGGGA
TGCTGGACGACTTTCTGGAACTTCTTCCCT

TCCAGACCCTGATGGGTCGCCTGGAAGATG
AGGTCTGGGACTACCCAGCGGACCTTCTAC

GTTCACCACGCACTGGTCAGATCTTC 3'
CAAGTGGTGCGTGACCAGTCTAGAAG

constitutes a part of the DNA sequence which codes for the human growth hormone.
36. Human nerve growth factor fused protein represented by the following formula,

```
                                                  10
      met  phe  pro  thr  ile  pro  leu  ser  arg  leu
                                                  20
      phe  asp  asn  ala  met  leu  arg  ala  his  arg
                                                  30
      leu  his  gln  len  ala  phe  asp  thr  tyr  gln
                                                  40
      glu  phe  glu  glu  ala  tyr  ile  pro  lys  glu
                                                  50
      gln  lys  tyr  ser  phe  leu  gln  asn  pro  gln
```

```
                                                    60
        thr   ser   leu   cys   phe   ser   glu   ser   ile   pro

                                                    70
        thr   pro   ser   asn   arg   glu   gln   thr   gln   gln

                                                    80
        lys   ser   asn   leu   glu   leu   leu   arg   ile   ser

                                                    90
        leu   leu   leu   ile   gln   ser   trp   leu   glu   pro

                                                    100
        val   gln   phe   leu   arg   ser   val   phe   ala   asn

                                                    110
        ser   leu   val   tyr   gly   ala   ser   asp   ser   asn

                                                    120
        val   thr   asp   leu   leu   lys   asp   leu   glu   glu

                                                    130
        gly   ile   gln   thr   leu   met   gly   arg   leu   glu

                                                    140
        asp   gly   ser   pro   arg   thr   gly   gln   ile   phe


        Val   Pro   Arg   SER   SER   SER   HIS   PRO   ILE   PHE
                     10
        HIS   ARG   GLY   GLU   PHE   SER   VAL   CYS   ASP   SER
                     20
        VAL   SER   VAL   TRP   VAL   GLY   ASP   LYS   THR   THR
                     30
        ALA   THR   ASP   ILE   LYS   GLY   LYS   GLU   VAL   MET
                     40
        VAL   LEU   GLY   GLU   VAL   ASN   ILE   ASN   ASN   SER
```

32

|     |     | 50  |     |     |     |     |     |     |     |
| --- | --- | --- | --- | --- | --- | --- | --- | --- | --- |
| VAL | PHE | LYS | GLN | TYR | PHE | PHE | GLU | THR | LYS |
|     |     | 60  |     |     |     |     |     |     |     |
| CYS | ARG | ASP | PRO | ASN | PRO | VAL | ASP | SER | GLY |
|     |     | 70  |     |     |     |     |     |     |     |
| CYS | ARG | GLY | ILE | ASP | SER | LYS | HIS | TRP | ASN |
|     |     | 80  |     |     |     |     |     |     |     |
| SER | TYR | CYS | THR | THR | THR | HIS | THR | PHE | VAL |
|     |     | 90  |     |     |     |     |     |     |     |
| LYS | ALA | LEU | THR | MET | ASP | GLY | LYS | GLN | ALA |
|     |     | 100 |     |     |     |     |     |     |     |
| ALA | TRP | ARG | PHE | ILE | ARG | ILE | ASP | THR | ALA |
|     |     | 110 |     |     |     |     |     |     |     |
| CYS | VAL | CYS | VAL | LEU | SER | ARG | LYS | ALA | VAL |
| 118 |     |     |     |     |     |     |     |     |     |
| ARG. |     |     |     |     |     |     |     |     |     |

FIG. 1

H2NCH2—⟨◯⟩—Ⓟ

DMTrO—⌐B—OCO(CH2)2CO—O—C6Cl5 (pentachlorophenyl)

DMTrO—⌐B—OCO(CH2)2CO—NHCH2—⟨◯⟩—Ⓟ

1) BSA

2) DMTrO—⌐B—OPO(=O)(OAr)—⌐B—OPŌ(=O)(OAr) Et3N⁺H . MSNT

3) Ac2O—DMAP

7 Repetitions

DMTrO—⌐B—OPO(=O)(OAr)—⌐B—OP(=O)(OAr)—(O—⌐B—OPO(=O)(OAr)—⌐B—OP(=O)(OAr))₆—O—⌐B—OCO(CH2)2CO—NHCH2—⟨◯⟩—Ⓟ

1) TMG PAO
2) conc. NH4OH
3) 80% AcOH

HO—⌐B'—OP(=O)(O⁻)—O—(⌐B'—OP(=O)(O⁻)—O)₁₃—⌐B'—OH

FIG. 2

Fig. 3

In Fig. 3:

"Trp $^P/_O$" = tryptophan promotor/operator

"Met-hGH (A,B)" = DNA sequence coding for Met and coding for A,B of human growth hormon

"Ile, Glu, Gly, Arg" = DNA sequence coding for Ile, Glu, Gly, Arg

"β-NGF" = DNA sequence coding for β-NGF

"Amp$^r$" = DNA sequence coding for ampicillin resistance

"Ori" = DNA sequence coding for the initiation point of replication

"pBR 322" = a plasmid disclosed in "Sutliffe, J. G." (1979) (Cold Spring Harbor Symposium, 43)

Fig. 4

In Fig. 4:

"Val, Pro, Arg"  = DNA sequence coding for Val, Pro, Arg

* Synthetic DNA fragment I (see Examples 1 and 2)

## DOCUMENTS CONSIDERED TO BE RELEVANT

EP 89102795.5

| Category | Citation of document with indication, where appropriate, of relevant passages | Relevant to claim | CLASSIFICATION OF THE APPLICATION (Int. Cl.4) |
|---|---|---|---|
| X | EP - A1 - 0 121 338 (GENENTECH, INC.)<br><br>* Fig. 5 * | 36 | C 12 N 15/00<br>·C 12 N 1/20<br>C 12 P 21/00 |
| A | * Claims; fig. 5 *<br><br>-- | 1-35 | C 07 K 13/00 |
| D,X | NATURE, vol. 303, no. 5920, June 23, 1983, New York, London<br><br>A.ULLRICH et al. "Human ß-nerve growth factor gene sequence highly homologous to that of mouse" pages 821-925 | | //(C 12 N 1/20,<br>C 12 R 1:19) |
| | * Page 823; fig. 2 * | 36 | |
| D,A | * Totality *<br><br>-- | 1-16 | |
| D,A | PATENT ABSTRACTS OF JAPAN, unexamined applications, C field, vol. 11, no. 38, February 4, 1987<br><br>THE PATENT OFFICE JAPANESE GOVERNMENT<br>page 164 C 401 | 1-35 | TECHNICAL FIELDS SEARCHED (Int. Cl.4) |
| | * Kokai-no. 61-205 485 (EIKO OTSUKA) *<br><br>---- | | C 12 N<br>C 12 P<br>C 07 K |

The present search report has been drawn up for all claims

| Place of search | Date of completion of the search | Examiner |
|---|---|---|
| VIENNA | 09-05-1989 | WOLF |